# EUROPEAN PATENT APPLICATION

(11) **EP 0 563 876 A2**
(43) Date of publication of application: **06.10.1993**
(21) Application number: 93105235.1
(22) Date of filing: 30.03.1993
(51) Int. Cl.: A61K 9/16, A61K 9/14, A61K 7/00

(54) **Biodegradable microbeads for the pharmaceutical and cosmetic uses**

(30) Priority: 01.04.1992 IT MI920785
(71) Applicant: INVERNI DELLA BEFFA S.P.A., I-20141 Milano (IT)
(72) Inventor: Forni, Flavio, I-20141 Milano (IT); Cameroni, Riccardo, I-20141 Milano (IT); Furiosi, Giuseppe, I-20141 Milano (IT); Pifferi, Giorgio, I-20141 Milano (IT)
(74) Representative: Bianchetti, Giuseppe

(57) **Abstract**

Biodegradable microbeads, suitable for the preparation of formulations which can be administered by the oral, parenteral and topical routes, are obtained treating natural proteins (gelatin, albumin, casein) con glyceraldehyde as the cross-linking agent, optionally already in the presence of active ingredients.

## Description

The present invention relates to biodegradable microbeads suitable for the preparation of formulations which can be administered by the oral, parenteral and topical routes.

Multiparticellar pharmaceutical forms, such as microbeads, allow a better control of the release of the active ingredients compared with the monolytic formulations. In fact, by the oral route, for example, the release from said systems is less affected by the passage time through the intestinal tract and by any food present.

The microbeads of the invention were prepared according to suitable procedures starting from heterogeneous mixtures of biocompatible proteins soluble in agueous media, such as gelatin, albumin e casein. Gelatin obtained either by controlled hydrolysis of collagen or by heating tendons, skins, ligaments and bones in water turned out to be particularly suited as a starting product.

In the pharmaceutical field, the use of a biocompatible polymer, preferably naturally occurring, shows remarkable advantages compared with the normal synthetic polymers. Thus, for instance, gelatin is perfectly biodegradable, being digested in the human gastrointestinal tract.

It is known that the physico-chemical characteristics (such as solvent solubility) and degradation rate of biodegradable polymers can be modulated by cross-linking reactions. Such processes can be carried out by treatment with suitable cross-linking agents such as: formaldehyde, glutaraldehyde, hexamethylenediisocyanate, dichloro- or trichloro-triazine [A. Courts, UK 1,191,873 (1970)]. The preparation of formaldehyde cross-linked gelatin microbeads for the pharmaceutical use is described by N. Tanaka et al., J. Pharm. Sci., 52, 664 (1963) and by C. Chemtob et al., Drug Development and Industrial Pharmacy, 14, 1359 (1988). The preparation of glutaraldehyde cross-linked gelatin microbeads is described by G. Raymond et al., Drug Development and Industrial Pharmacy 16, 1025 (1990).

An important limitation to the pharmaceutical and cosmetic uses of the polymers cross-linked with the reactives used up to now resides in the toxicity of said reactives which can remain in form of residues in the microbeads or can be released during the depolymerization processes.

In the case of glutaraldehyde cross-linked natural polymers, cytotoxic effects and other adverse biological reactions have been described [D.P. Speer et al., J. of Biomed. Mater. Res., 14, 753 (1980)].

A basic aspect of the present invention resides in the use of glyceraldehyde as cross-linking agent, which is present in the laevo form in human body as a product from fructose metabolism due to aldolase action on fructose-1-phosphate. Glyceraldehyde is then phosphorylated by triosokinase to glyceraldehyde-3-phosphate, thus entering the glycolysis cycle.

Glyceraldehyde is known to exist, besides in the laevo form, also in the dextro form and as racemate. The present invention comprises the use of both the racemate and the single enantiomers as well as the mixtures thereof in ratios different from 1:1.

The cross-linking potential of aldotrioses, including glyceraldehyde, was studied towards casein [K.D. Schwenke et al., Nahrung, 20, 895 (1976)] and proteins in general [A.S. Acharya et al., Biochemistry, 27, 4522 (1988)] only as far as the reaction mechanism is concerned, but in these works no descriptions about the preparation of a cross-linked polymer can be found, let alone characterizations and general applications thereof, particularly pharmaceutical and cosmetic applications.

In the cross-linking reaction, glyceraldehyde can react with the reactive groups of the protein, particularly with the amino, guanidino, alcohol and thiol groups. A schematic representation of the condensation with a protein amino group could be the following one: 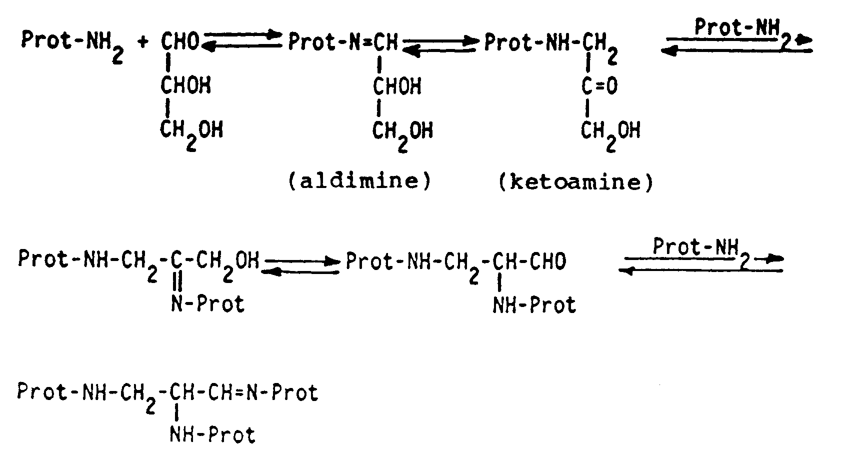
and, with two protein groups: 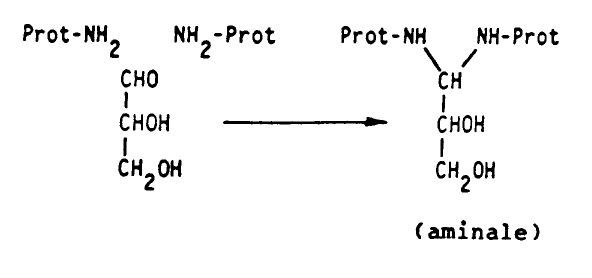
The scope of the present Patent application, of course, is not limited by the true nature of the cross-linking mechanism neither by the one of the protein functional groups involved in cross-linking itself.

A further aspect of the present invention is provided by the manufacture of microbeads (micropellets) of gelatin or other glyceraldehyde cross-linked natural proteins, to obtain a novel biodegradable polymer having, inter alia, bioadhesive properties.

Form this point of view, particularly advantageous is the intranasal formulation of medicaments that cannot be administered orally, since they can be degraded in the gastrointestinal tract, or alternatively to the parenteral administration. Moreover, the polymer bioadhesiveness allows to prepare other topical pharmaceutical forms, for example for the rectal or vaginal uses.

The preparation of the cross-linked gelatin microbeads can be carried out according to the scheme of Process 1, emulsifying the gelatin aqueous solution (optionally also containing one or more active ingredients, dissolved or suspended) with an oily phase consisting of suitable mineral, vegetable or semisynthetic oils. Thereafter, dehydration is carried out by adding a water-miscible solvent, such as acetone, and the not cross-linked microbeads are prepared, which are subsequently cross-linked (i.e. subjected to cross-linking) with a glyceraldehyde (G.A.) aqueous solution. 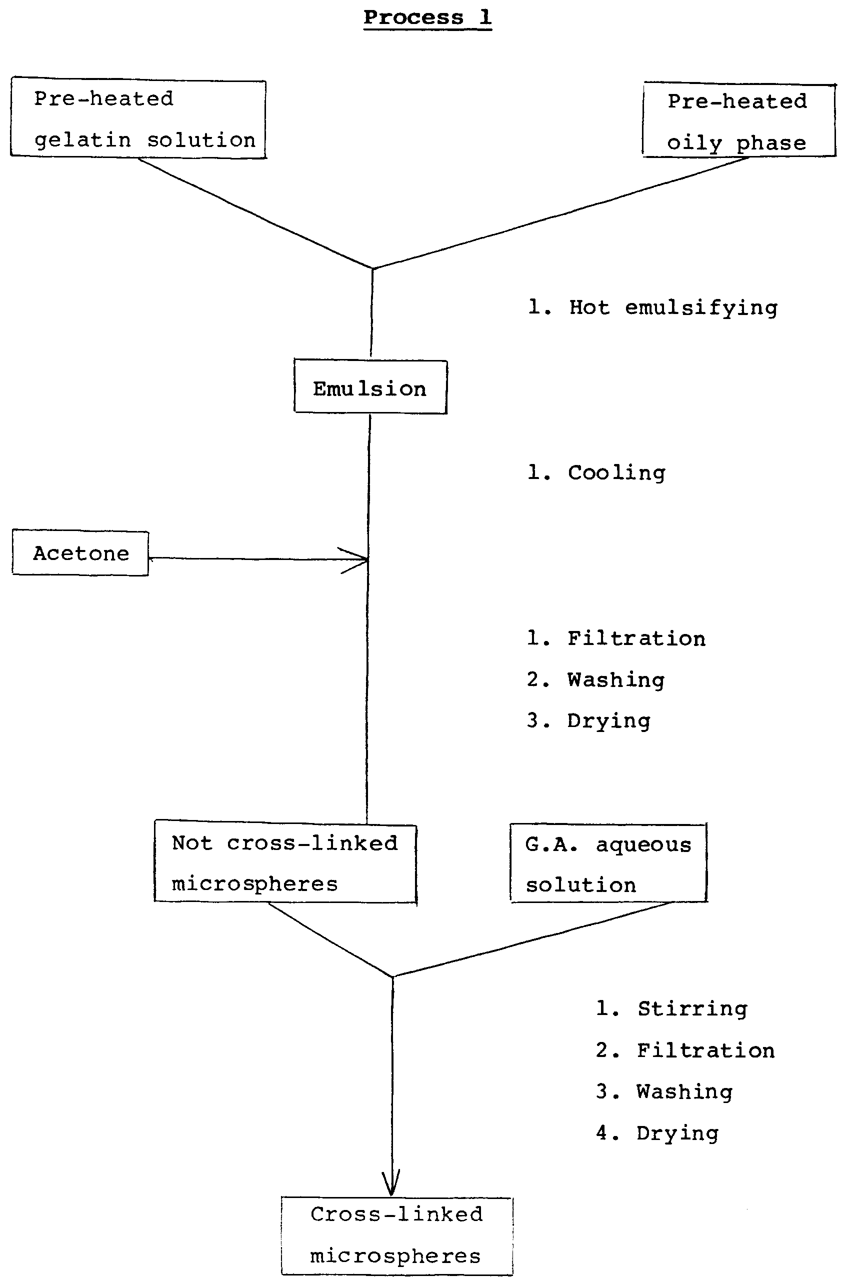
The cross-linking can also be carried out directly by adding glyceraldehyde after emulsifying, without separating the not cross-linked microbeads. The solvent dehydration can be carried out either after the addition of the cross-linking agent as described in the scheme of Process 2, or before the addition. Also according to this process, the gelatin aqueous solution can contain one or more active ingredients, dissolved or suspended. 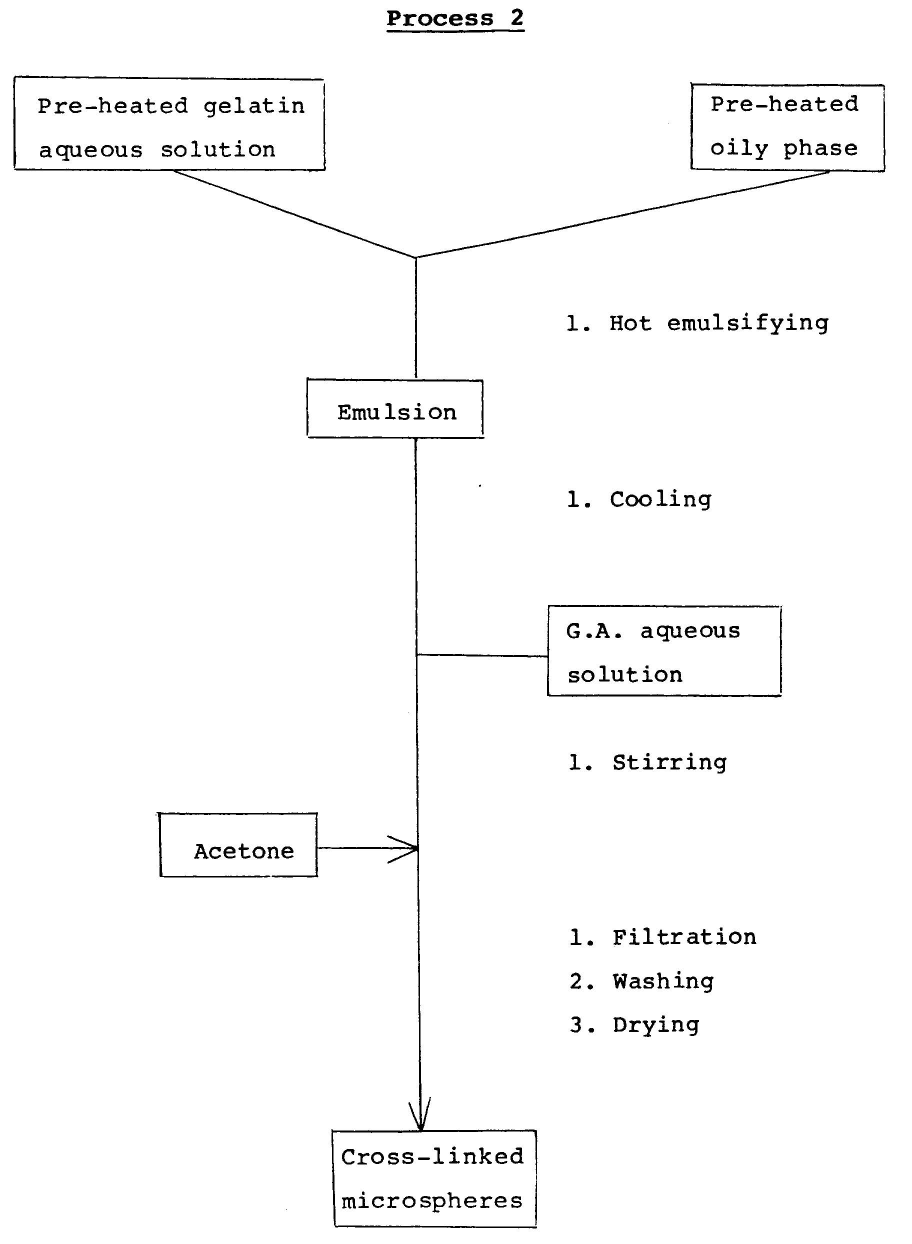
A third alternative of the preparation of the cross-linked microbeads consists in carrying out the emulsifying already in the presence of glyceraldehyde and subsequently the solvent dehydration step according to the scheme of Process 3. Also according to this process, the gelatin aqueous solution can contain one or more active ingredients, dissolved or suspended. 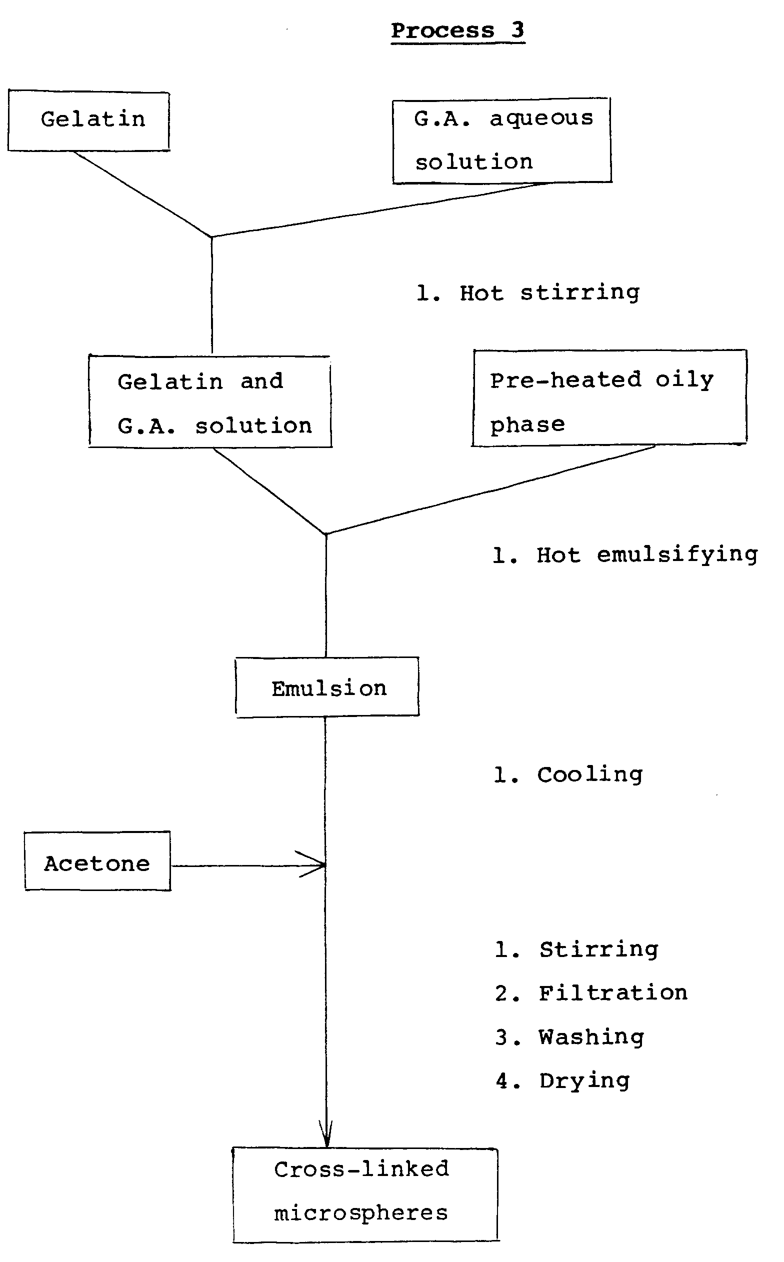
The thus cross-linked gelatin microbeads can be used for the preparation of oral, parenteral and topical pharmaceutical formulations. Therefore the incorporation of suitable concentrations of active ingredients for the therapeutic or cosmetic uses in said microbeads, was studied and carried out, according to 2 basic processes:
a) incorporation of the active ingredients during the process for the preparation of the microbeads (as mentioned above),
b) adsorption of a solution or suspension of the active ingredients in the previously cross-linked microbeads, in case the active ingredients are incompatible with glyceraldehyde or with the dehydration solvents.

According to another aspect of the present invention pharmaceutical grade gelatins can be used, obtained either by acid (type A) or basic (type B) hydrolysis, having a gelling strength ranging from 60 to 300 Blooms and a molecular weight from 15,000 to 250,000 Daltons. Preferably, a gelatin of 208 Bloom, having average molecular weight 176,000 Daltons and viscosity of the Bloom solution at 60°C of 4.07 mPa sec. (By "gelling strength", it is meant the weight necessary to cause a decrease of 4 mm of an aqueous gel containing 6.67% (w/w) of the tested gelatin, thermostatized at 10°C, using a gel meter with a 12.7 mm diameter piston).

The oily phase used for emulsifying can be selected from mineral oils, such as liquid paraffin (Mineral oil), vegetable oils such as saturated and unsaturated oils obtained from maize, sunflower, corn, soy, line, peanut seed and from olive, or semisynthetic oils, particularly Miglyol^{R} ("Fractionated Coconut Oil", see British Pharmacopoeia 1988).

Emulsifying can be carried out mixing with a turboemulsifier operating at 5,000 to 20,000 rpm, a gelatin aqueous solution of 10-40% (w/v) concentration with an oily phase equivalent to 2-10 folds the volume of the aqueous phase. The oily phase and the gelatin aqueous solution must be pre-heated to temperatures ranging from 40° to 80°C. The dehydration step can be performed at a temperature from 0° to 30°C, preferably at 5°C, with water-miscible solvents such as ethanol, isopropanol and acetone. Acetone or isopropanol in volumes of about 2-10 folds the aqueous phase were advantageously used.

The cross-linking of the microbeads can be performed in aqueous or aqueous-organic solutions, using both the laevo- and the dextro- glyceraldehyde, or the racemic form, or mixtures of the single enantiomers in ratios different from 1:1.

The concentration of the used cross-linking agent, the temperature and the cross-linking time are parameters affecting the physico-chemical characteristics of the final microbeads and they can suitably be changed to optimize both said characteristics and the release profile of the active ingredient contained in the microbeads.

Particularly, the glyceraldehyde concentration in the cross-linking solution can be comprised from 0.1 to 3% w/v, the cross-linking times can vary from 0.5 to 24 hours and the temperatures range between 0° and 60°C.

The glyceraldehyde to gelatin weight ratio can range from 1:2 to 1:100, preferably from 1:5 to 1:30.

The resulting gelatin microbeads form a flowing light yellow powder, consisting of small particles of spherical shape as evidenced at the microscope examination.

The granulometric analysis of the product, carried out by means of a laser granulometer, evidenced that the average size of the particles can be changed from 10 to 500 µm, depending on the experimental conditions. Particularly, granulometry can optionally be reduced adding suitable biocompatible surfactants (such as phosphatidylcholine, sorbitan esters and the like). The resulting microbeads are insoluble in water even at 37°C, wherein they swell, contrarily to the starting gelatin, which is highly soluble. The content in residual glyceraldehyde, determined by a specific chromatographic method, is very low and it can be measured at a level of parts per million.

The biodegradability of the microbeads was evaluated both in vitro and in vivo.

A turbidimetric method was used in vitro, which consists in adding scalar concentrations of a proteolytic enzyme (for example trypsin) to a microbeads suspension in phosphate buffer at pH 7.4 at 37°C. The biodegradation was evidenced from the increase in the transmittance percent values.

In vivo a microbead suspension in 0.5% methocel is injected subcutaneously in the rat. Thereafter, the animals are killed 30, 60 and 120 minutes after the injection, recovering the injected suspension. The observation under optical microscope reveals the decomposition and progressive degradation of the microbeads. The examination of the injection site evidenced no macroscopic changes of the subcutaneous tissue, confirming that the microbeads under test are biodegradable and biocompatible.

The microbeads according to the invention release the active ingredient (or the active ingredients) through a double mechanism: diffusion and/or biodegradation of the polymer. Said microbeads can be administered either orally, if the nature of the active ingredient allows it, or parenterally, in alternative to the conventional injectable formulations, improving the patient compliance thanks to a decreased number of administrations, or topically.

The cross-linked microbeads, depending on the biodegradability and biocompatibility, can be administered as long-lasting medications in the gingival pockets, for example in case of paradentosis. Moreover, said microbeads can act a a medicament reservoir (for example antibiotics) to be introduced into the soft tissues and bones for the treatment of osteomyelitis or of infected wounds.

The microbeads can optionally be coated with pharmaceutical polymers to modulate the diffusion process by means of the restrictive action of the superficial coating. The use of coating substances which can dissolve either in different areas of the gastrointestinal tract (depending on pH and/or on the present enzymes) or after a preset time (depending on the thickness) allows said coated microbeads to be used both in a space- and time-aimed way.

The topical administration of cross-linked gelatin microbeads, moreover, can be advantageous to prolong the activity of medicaments directed at the cutis level. In fact, the presence of the medicament inside a matrix reservoir allows to reduce the administrations, particularly in the presence of an occlusive bandage.

Finally, the mechanical action of the microbeads can be exploited for the cosmetic use, said microbeads being incorporated in suitable ointments and anhydrous gels, intended to exert a peeling effect or as a reservoir of vegetable extracts or of other product exerting a moisturizing, tonic, protecting and/or decongestant on epidermis and appendages thereof.

The following examples further illustrate the invention.

### Example 1: Cross-linked microbeads according to process 1

Gelatin (3 g) is dissolved in 10 ml of distilled H₂O at 60°C under stirring and 20 ml of liquid paraffin, pre-heated to the same temperature, are added under stirring. The mixture is emulsified and cooled under stirring to a temperature of 5°C. When this temperature is reached, 30 ml of acetone cooled to a temperature of 5°C are added. The mixture is stirred for 15 minutes, then it is filtered, washed with acetone and dried under vacuum. The not cross-linked particles are placed into a flask containing 10 ml of a 3% w/v DL-glyceraldehyde aqueous solution (0.3 g; 3.33 mmol), which thereafter can optionally be added with 20 ml of acetone, cooled to 5°C. The mixture is stirred at 5°C for 10 minutes, filtered, washed with acetone and dried under vacuum.

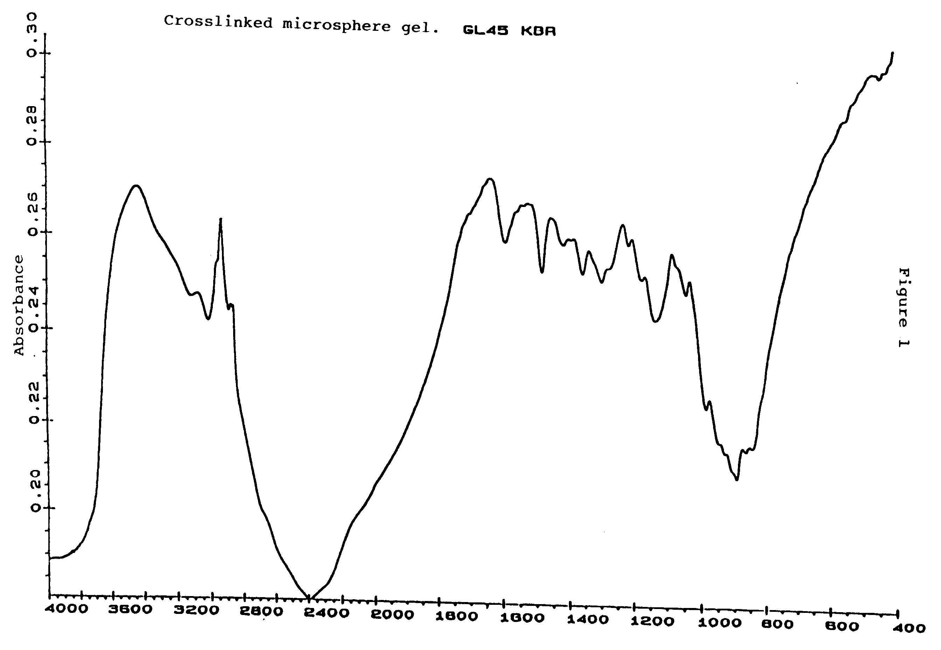

2.65 g of straw-yellow cross-linked microbeads are obtained. In Fig. 1, the IR spectrum of the product is reported, recorded in KBr pellets.

### Example 2: Cross-linked microbeads according to process 2

Gelatin (3 g) is dissolved in 10 ml of distilled H₂O at 60°C under stirring and 20 ml of liquid paraffin pre-heated to the same temperature are added under stirring. The mixture is emulsified and cooled under stirring to room temperature. Thereafter, 5 ml of a 3% w/v DL-glyceraldehyde aqueous solution (0.15 g; 1.665 mmol) are added and the stirring is continued at room temperature for 5 h. 40 ml of acetone are added and stirring is continued for 10 minutes. The mixture is filtered, washed with acetone and dried under vacuum.

2.8 g of cross-linked microbeads are obtained.
Note: acetone can be added before the glyceraldehyde solution.

### Example 3: Cross-linked microbeads according to process 3

Gelatin (3 g) is dissolved in 10 ml of a 2% w/v DL-glyceraldehyde aqueous solution (0.2 g; 2.22 mmol) at 60°C, under stirring, and 20 ml of liquid paraffin pre-heated to the same temperature are added under stirring. The mixture is emulsified and cooled under stirring to a temperature of 5°C. When this temperature is reached, 10 ml of acetone cooled to a temperature of 5°C are added and stirring is continued with cooling for 24 h. The mixture is filtered, washed with acetone and dried under vacuum.

2.9 g of cross-linked microbeads are obtained.

### Example 4: Microbeads containing clonidine hydrochloride cross-linked according to process 1

Gelatin (3 g) is dissolved, at 60°C and under stirring, in 10 ml of distilled H₂O in which 0.5 g of clonidine hydrochloride were previously dissolved at room temperature. 30 ml of liquid paraffin pre-heated to the same temperature are added under stirring. The mixture is emulsified for 10 minutes and cooled to a temperature of 5°C, under stirring. Thereafter, 40 ml of acetone are added, cooled to a temperature of 5°C. Stirring is continued for 15 minutes. The mixture is filtered, washed with acetone and dried under vacuum.

The microbeads are added with 10 ml of a 3% w/v DL-glyceraldehyde aqueous solution (0.3 g; 3.33 mmol), added with 20 ml of acetone, cooled a 5°C. The mixture is stirred at 5°C for 1 h, then it is filtered, washed with acetone and dried under vacuum.

2.64 g of microbeads containing 3% of medicament are obtained (microbeads yield: 88%; loading yield: 16%).

### Example 5: Microbeads containing clonidine hydrochloride cross-linked according to process 3

Gelatin (3 g) is dissolved, at 60°C and under stirring, in 10 ml of a 2% w/v DL-glyceraldehyde aqueous solution (0.2 g; 2.22 mmol), in which 0.5 g of clonidine hydrochloride had previously been dissolved at room temperature. After that, 20 ml of liquid paraffin pre-heated to the same temperature are added under stirring. The mixture is emulsified for 10 minutes, then cooled under stirring to a temperature of 5°C. Then 30 ml of acetone cooled to 5°C are added and stirring is continued for 4 h. The mixture is filtered, washed with acetone and dried under vacuum.

2.80 g of cross-linked microbeads are obtained, containing about 7% of medicament (microbeads yield: 92%; loading yield: 40%).

### Example 6: Cross-linked microbeads loaded by adsorption with clonidine hydrochloride

Cross-linked microbeads are prepared according to process 1, starting from 3 g of gelatin, then they are washed with acetone and dried under vacuum. The resulting microbeads are placed into a glass column wherein a 1% clonidine hydrochloride aqueous solution is recycled, until loading has taken place. The the microbeads are separated by filtration and dried under vacuum.

2.70 g of cross-linked microbeads are obtained, containing about 10% of medicament (microbeads yield: 90%; loading yield: 27%).

### Example 7: Capsules of cross-linked microbeads containing salbutamol sulfate

The following ingredients are dry mixed:
100 mg of cross-linked microbeads containing 2.4 mg of salbutamol sulfate
52 mg of lactose
1.6 mg of magnesium stearate
4.8 mg of talc
1.6 mg of colloidal silica.

The mixture is then distributed in hard-gelatin capsules of size 2.

### Example 8: injectable preparation containing cross-linked microbeads of clonidine hydrochloride

Cross-linked sterile microbeads (15 mg) with sizes of 10-50, µm containing 2% of clonidine hydrochloride are mixed with 100 mg of dextrose. The mixture is distributed in vials for the extemporary use after suspension in a suitable sterile liquid.

### Example 9: Anhydrous gel with cross-linked microbeads containing betamethasone 17-valerate

A gel is prepared containing :
35 g of polyethylene glycol 400
61 g of monoethyl ether diethylene glycol
2 g of carboxyvinyl polymer
in which the following ingredients are then dispersed:
2 g of cross-linked microbeads containing 5% betamethasone 17-valerate.

### Example 10: L-glyceraldehyde cross-linked gelatin microbeads

3 g of gelatin are dissolved in distilled water (10 ml) at 60°C under stirring, then liquid paraffin (30 ml) pre-heated to the same temperature is added thereto. The two phases are emulsified and cooled under stirring to a temperature of 5°C. When this temperature is reached, 30 ml of acetone cooled to 5°C are added and stirring is continued for 15 minutes. The mixture is filtered, washed with acetone and dried under vacuum. The solid microbeads are placed into a flask containing 10 ml of a 1% w/v L-glyceraldehyde aqueous solution (0.1 g; 1.11 mmol), which then can optionally be added with 20 ml of acetone cooled to 5°C, and the mixture is stirred at 5°C for 24 hours: the microbeads are filtered, washed with acetone and dried under vacuum.

### Example 11: D,L-glyceraldehyde cross-linked casein microbeads

2 g of casein are dissolved in 6 ml of 0.6 M sodium hydroxide at 60°C under stirring and then the solution is heated to 90-95°C for 1 minute. The casein inner phase is added to an outer phase consisting of liquid paraffin pre-heated to 60°C, and stirring is continued. The mixture is cooled to room temperature and 5 ml of a 3% w/v D,L-glyceraldehyde aqueous solution are added (0.15 g; 1.66 mmol), keeping stirring at room temperature for 3 hours. Acetone is added, stirring for 15 minutes. The microbeads are filtered, washed with acetone and dried under vacuum.

### Example 12: D,L-glyceraldehyde cross-linked casein microbeads

4 g of casein are dissolved in 0.6 M sodium hydroxide (6 ml) at 60°C under stirring and the solution is heated to 90-95°C for 1 minute. The outer phase, consisting of liquid paraffin pre-heated to 60°C, is added to an inner phase while stirring. After cooling to room temperature, 5 ml of a 3% w/v D,L-glyceraldehyde aqueous solution (0.15; 1.66 mmol) are added, and stirring is continued at room temperature for 3 hours. Acetone is added, stirring for 15 minutes. The microbeads are filtered off, washed with acetone and dried under vacuum.

### Example 13: Cross-linked microbeads loaded by adsorption with rifamycin SV sodium salt

The cross-linked microbeads are prepared according to the process 1, starting from 6 g of gelatin, by cross-linking with a 0.5% w/v D,L-glyceraldehyde aqueous solution, stirring at 5°C for 1 h. The microbeads are washed with acetone and dried under vacuum. 18 ml of a 0.5% rifamycin SV sodium salt aqueous solution are adsorbed on 4 g of the obtained microbeads. When the solution is completely adsorbed, it is frozen and the obtained humid mass is freeze-dried.

3.6 g of cross-linked microbeads are obtained, containing 23% of medicament (microbeads yield: 90%, loading yield: 90%).

### Example 14: Cross-linked microbeads loaded by adsorption with whortleberry antocyanosids

The cross-linked microbeads are prepared according to the process 1, starting from 6 g of gelatin, by cross-linking with a 0.5% w/v D,L-glyceraldehyde aqueous solution, stirring at 5°C for 1 h. The microbeads are washed with acetone and dried under vacuum. 20 ml of a 4% whortleberry antocyanosids aqueous solution are adsorbed on 3 g of the obtained microbeads. When the solution is completely adsorbed, it is frozen and the obtained humid mass is freeze-dried.

2.7 g of cross-linked microbeads are obtained, containing 27% of medicament (microbeads yield: 90%, loading yield: 90%).

## Claims

1. Biodegradable and biocompatible microbeads consisting of proteins, or mixtures thereof, cross-linked with glyceraldehyde.

2. Microbeads according to claim 1, consisting of natural proteins, or mixtures thereof, cross-linked with glyceraldehyde.

3. Microbeads according to claims 1 and 2, consisting of proteins selected from gelatin, albumin and casein, cross-linked with glyceraldehyde.

4. Microbeads according to claim 3, consisting of gelatin of pharmaceutical grade cross-linked with glyceraldehyde.

5. Microbeads according to claims 1-4, characterized in that glyceraldehyde is (±)-, (-)- or (+)-glyceraldehyde or mixtures of the two enantiomers in ratios different from 1:1.

6. Microbeads according to the above claims, containing one or more pharmaceutically active ingredients.

7. Microbeads according to the above claims, containing one or more ingredients for the cosmetic use.

8. Microbeads according to the above claims, coated with polymers for the pharmaceutical use.

9. Pharmaceutical compositions containing or consisting of the microbeads according to claims 5, 6 and 8.

10. Pharmaceutical compositions according to claim 9, for the oral, parenteral, topical or intranasal administrations.

11. A process for the preparation of biodegradable microbeads according to claims 1-5, in which process:
a) a protein aqueous solution is emulsified with an oily phase consisting of mineral, vegetable or semisynthetic oils, b) the mixture is dehydrated by addition of a water-soluble solvent, c) the not cross-linked microbeads are separated, d) said microbeads are cross-linked with a glyceraldehyde solution; said glyceraldehyde solution being optionally added to the emulsion before dehydration step b), in which case the microbeads are separated only after cross-linking; or said glyceraldehyde solution being added to the protein aqueous solution before emulsifying, in which case also the microbeads being separated only after cross-linking.

12. A process according to claim 11, in which the protein is selected from gelatin, albumin and casein.

13. A process according to claim 12, in which gelatin of pharmaceutical grade is used as the protein.

14. A process according to claim 13, in which a gelatin aqueous solution of pharmaceutical grade is used, in a concentration from 10 to 40% (w/v).

15. A process according to claims 11-14, in which acetone or isopropanol is used as the water-miscible solvent for the dehydration of the emulsion.

16. A process according to claims 11-15, in which the weight ratio of glyceraldehyde to protein ranges from 1:2 to 1:100.

17. A process according to claim 16, in which the weight ratio of glyceraldehyde to protein ranges from 1:5 to 1:30.

18. A process according to claims 11-17, for the preparation of biodegradable microbeads containing one or more pharmaceutically or cosmetically active ingredients according to claims 6 and 7, in which process a glyceraldehyde aqueous solution contains one or more pharmaceutically or cosmetically active ingredients dissolved or suspended therein.

19. A process according to claims 11-18 for the preparation of biodegradable microbeads containing one or more pharmaceutically or cosmetically active ingredients according to claims 6 and 7, in which process the obtained cross-linked microbeads are treated with a solution or suspension of one or more pharmaceutically or cosmetically active ingredients.
